# FASCICULE DE BREVET EUROPEEN

(11) **EP 2 327 366 B1**
(45) Date de publication et mention de la délivrance du brevet: **14.03.2012**
(21) Numéro de dépôt: 10178579.8
(22) Date de dépôt: 23.09.2010
(51) Int. Cl.: A61B 18/14, A61N 1/05

(54) **Nécessaire de perçage du septum cardiaque et d'implantation d'une sonde transseptale, notamment une sonde de détection/stimulation d'une cavité gauche du coeur**
Besteck zum Bohren des Herzseptums und Implantieren einer transseptalen Sonde, insbesondere einer Sonde zum Feststellen/Stimulieren einer Vertiefung links vom Herzen
Kit for piercing the cardiac septum and implanting a transseptal probe, in particular a probe for detection/stimulation of a left cavity of the heart

(30) Priorité: 30.11.2009 FR 0958504
(43) Date de publication de la demande: 01.06.2011
(73) Titulaire: Sorin CRM SAS, 92143 Clamart Cedex (FR)
(72) Inventeur: Ollivier, Jean-François, 91190, Villiers le Bacle (FR)
(74) Mandataire: Dupuis-Latour, Dominique

(56) Documents cités:
- EP-A1- 0 591 053
- EP-A1- 1 516 644
- US-A- 6 016 809
- US-A- 6 086 582
- US-A1- 2002 188 340
- US-A1- 2005 234 444
- US-B1- 6 565 562

## Description

L'invention concerne un nécessaire de perçage du septum cardiaque et d'implantation d'une sonde transseptale.

Elle s'applique tout particulièrement à l'implantation d'une sonde de détection/stimulation d'une cavité gauche du coeur permettant de surveiller en continu le rythme cardiaque et délivrer si nécessaire au coeur des impulsions électriques de stimulation et/ou de resynchronisation en cas de trouble du rythme détecté par le dispositif.

L'invention n'est toutefois pas limitée à la pose de telles sondes de détection/stimulation. Elle s'applique également à la pose de sonde de défibrillation et, de façon encore plus générale, à toute technique chirurgicale invasive nécessitant la traversée du septum cardiaque, par exemple pour procéder à des investigations cliniques dans l'une ou l'autre des cavités gauches. Il peut s'agir également de procédure d'angioplastie, notamment pour des opérations de valvuloplastie mitrale.

Pour la stimulation des cavités droites du coeur, il suffit d'implanter une sonde endocavitaire par le réseau veineux périphérique droit. En revanche, pour stimuler les cavités gauches, la situation est plus complexe et la solution la plus souvent retenue consiste à introduire la sonde par l'oreillette droite puis dans le réseau coronarien via l'ostium du sinus coronaire.

Cette technique d'implantation n'est cependant pas toujours réalisable, notamment lorsque la conformation du sinus coronaire est trop accidentée, ou en case de thrombose.

Une autre solution, dite "approche transseptale", consiste à passer la sonde à travers la cloison interauriculaire ou interventriculaire, ou "septum cardiaque", pour stimuler la cavité gauche - auriculaire et/ou ventriculaire, selon la configuration et le placement de la sonde.

Cette procédure, telle qu'elle est mise en oeuvre actuellement, présente cependant des risques opératoires élevés, notamment de perforation accidentelle de l'aorte, ou encore de dissection des parois de l'oreillette droite par un mouvement rotatoire inopiné de l'aiguille.

En tout état de cause, cette technique est très délicate à mettre en oeuvre et requiert une grande habileté du chirurgien qui doit, pour pouvoir traverser le septum, s'assurer toujours du positionnement parfait sur la paroi, la traversée du septum ne devant être entreprise que s'il ne subsiste aucun doute sur la position de l'aiguille.

Le EP 1 516 644 A1 décrit un nécessaire de perçage permettant de résoudre ces difficultés.

La technique proposée consiste à mettre en oeuvre un guide de perçage constitué d'une sonde pourvue à son extrémité distale d'une vis hélicoïdale destinée à être vissée dans la paroi du septum à l'emplacement du site de perforation choisi. Une fois la vis ancrée en place, le praticien introduit dans la lumière interne de la sonde un cathéter-guide à l'intérieur duquel est emmanché un mandrin perceur. Ce dernier est progressivement enfoncé dans le cathéter-guide jusqu'à perforer le septum, tout en étant guidé dans sa progression par la tête de sonde dont l'ancrage en place est assuré grâce à la vis hélicoïdale, ce qui permet de sécuriser la progression du mandrin perceur. Une fois la paroi septale traversée de part en part, le cathéter-guide est poussé de manière à pénétrer dans l'orifice amorcé par le mandrin perceur, jusqu'à déboucher dans la cavité gauche. L'opération se poursuit par le retrait du mandrin perceur hors du cathéter-guide, puis l'extraction du guide de perçage (par dévissage de la vis et retrait). Seul reste alors en place le cathéter-guide, sur lequel est enfilé un dilatateur permettant d'agrandir l'orifice. Un cathéter principal est alors mis en place pour assurer une communication avec la cavité gauche et permettre l'introduction de la sonde de détection/stimulation, puis le positionnement de celle-ci jusqu'au site de stimulation endocavitaire choisi par le praticien dans la cavité gauche (en général l'oreillette).

Cette technique permet de réaliser une ponction parfaitement sécurisée de la paroi septale. En revanche, elle implique un nombre élevé d'objets différents à manipuler (sonde de guidage, mandrin perceur, cathéter-guide provisoire, dilatateur, cathéter-guide définitif, sonde de détection/stimulation, etc.). De plus, la technique de manipulation, si elle fait appel à des gestes opératoires usuels pour un praticien entraîné, n'en reste pas moins relativement délicate et longue à exécuter.

L'un des buts de l'invention est de proposer un nécessaire de perçage du septum cardiaque permettant de conserver le niveau de sécurité des techniques connues, tout en rassemblant en un seul et même objet les différents éléments nécessaires à sa mise en oeuvre.

Il serait certes possible d'ajouter des organes de découpe mécanique de type gouge ou analogue à une sonde de détection/stimulation traditionnelle, par exemple une sonde de type sonde rétractable à vis, qui constitue le système de fixation le mieux approprié à une fixation dans la paroi de la cavité gauche après traversée du septum.

Toutefois, le fait de laisser un organe de découpe, même dans une position escamotée, sur une sonde implantée de façon permanente dans le ventricule gauche semble un concept difficilement acceptable. Une alternative consistant à disposer autour de la sonde permanente un outil de découpe qui pourrait être ensuite retiré créerait un certain nombre de difficultés supplémentaires, du point de vue notamment de la sécurité d'emploi.

L'un des buts de l'invention est de remédier aux divers inconvénients, exposés plus haut, des techniques connues en proposant un nécessaire de perçage du septum cardiaque et de mise en place d'un dispositif transseptal, qui allie simplicité de mise en oeuvre et sécurité du perçage une fois le site d'intervention choisi, tout en réduisant au minimum le caractère invasif de cette intervention.

Un autre but de l'invention est de proposer un tel nécessaire de perçage qui puisse être mis en oeuvre par des techniques opératoires comparables à des techniques existantes (notamment la pose d'une sonde à vis par voie sous-clavière, comme on l'expliquera par la suite), auxquelles les praticiens sont rodés et qu'ils pourront adapter sans difficulté à une intervention de perçage du septum.

L'idée de base de l'invention consiste à combiner la technologie des sondes de stimulation à vis avec la technologie dite de "ponction RF" consistant à appliquer localement une énergie radiofréquence produite par un générateur électronique RF approprié, afin de créer une ouverture de très faible dimension dans le tissu cardiaque, notamment dans la paroi septale. Une telle ponction RF est opérée par application d'une énergie de faible puissance (5 à 25 W), pendant une brève période de temps (1 à 3 s) et sous haute tension (150 à 180 V), de manière à n'occasionner qu'un minimum de dommages collatéraux aux tissus environnants.

Cette technique de "ponction RF" est à distinguer de l"'ablation RF", qui consiste à appliquer une puissance élevée (35 à 50 W) pendant une durée prolongée (60 à 90 secondes) et sous basse tension (35 à 50 V). L'ablation RF aurait pour effet, dans l'application considérée ici, de créer une lésion plus étendue, avec destruction thermique des tissus environnants.

Le US 6 086 582 A décrit ainsi un dispositif pour traiter localement des sites ischémiques ou générateurs d'arythmies d'une cavité cardiaque, par délivrance *in situ* de doses de médicament. Cette délivrance est opérée à l'aide d'une sonde à vis que le praticien vient ancrer au site choisi à traiter. S'il souhaite en outre procéder à une ablation des tissus endommagés, il peut injecter localement une énergie RF délivrée par la vis d'ancrage, ce qui a pour effet de détruire les tissus (ablation) dans toute la région située autour de la vis. Mais cette technique ne conviendrait pas pour exécuter un perçage très précis et net (ponction) de la paroi cardiaque, a *fortiori* de à paroi septale, car la destruction difficilement contrôlable des tissus créerait un risque opératoire inacceptable. Elle ne peut donc être transposée telle quelle à une approche transseptale.

L'invention a pour but de s'affranchir de ces limitations, en proposant un nécessaire dédié au perçage du septum cardiaque et à l'implantation d'une sonde transseptale, qui procure les avantages exposés plus haut de simplicité, de sécurité et de caractère peu invasif.

Essentiellement, le nécessaire de l'invention comprend, en combinaison, une sonde de type sonde à vis et un générateur de ponction radiofréquence.

La sonde à vis est du type comportant, comme dans le US 6 086 582 A précité : un corps de sonde avec une gaine en matériau déformable ; côté proximal, un connecteur électrique de couplage à un boîtier de dispositif médical implanté ; côté distal, une tête de sonde avec une électrode comprenant une vis d'ancrage hélicoïdale saillante au moins partiellement conductrice, apte à pénétrer dans une paroi du coeur sous l'effet d'un mouvement de vissage imprimé à la tête de sonde depuis l'extrémité proximale de la sonde ; et un conducteur s'étendant le long de la gaine, reliant audit connecteur électrique l'électrode comprenant ladite vis d'ancrage. Le générateur de ponction radiofréquence est apte à être relié audit connecteur électrique pour permettre l'application contrôlée à la vis d'ancrage d'une énergie radiofréquence.

De façon caractéristique de l'invention, la paroi du coeur étant une paroi du septum cardiaque, la vis d'ancrage comprend une partie distale de traction électriquement isolée, prolongeant une partie proximale de découpe électriquement conductrice apte à permettre l'application contrôlée à la vis d'ancrage de l'énergie radiofréquence concurremment au mouvement de vissage imprimé à la tête de sonde. La sonde est ainsi apte à assurer le perçage de ladite paroi jusqu'à traversée du septum par la tête de sonde pour permettre une implantation de la sonde par voie transseptale.

La sonde peut notamment être une sonde de détection/stimulation d'une cavité gauche du coeur, ladite électrode de la tête de sonde étant une électrode de détection/stimulation.

La vis d'ancrage est soit une vis fixe prolongeant axialement la tête de sonde, soit une vis mobile rétractable dans un logement de la tête de sonde.

Dans le cas où la gaine du corps de sonde présente en torsion une rigidité insuffisante pour permettre la transmission sur toute sa longueur d'un mouvement de rotation imprimé depuis l'extrémité proximale de la sonde, le nécessaire comprend en outre un mandrin amovible, apte à être introduit dans une lumière du corps de sonde et mobile en translation à l'intérieur de cette lumière jusqu'à la tête de sonde et comportant des moyens de couplage en rotation avec la tête de sonde, ce mandrin présentant en torsion une rigidité suffisante pour permettre la transmission, sur toute sa longueur d'un mouvement de rotation imprimé depuis l'extrémité proximale du mandrin, pour le vissage de la vis d'ancrage.

Dans une variante de réalisation, le nécessaire comprend en outre un fil-guide perceur radiofréquence apte à permettre, après couplage au générateur de ponction radiofréquence, l'application contrôlée d'une énergie radiofréquence en un site de pré-ponction de la paroi du septum. La sonde est dans ce cas une sonde de type porteuse apte à être enfilée sur le fil-guide.

On va maintenant décrire un exemple de mise en oeuvre de l'invention, en référence aux dessins annexés où les mêmes références numériques désignent d'une figure à l'autre des éléments identiques ou fonctionnellement semblables.
La Figure 1 est une vue d'ensemble montrant les différents éléments du nécessaire de perçage de l'invention, dans une configuration correspondant au début de l'opération de perçage du septum.
La Figure 2 est homologue de la Figure 1, pour une variante avec utilisation d'un fil-guide RF.

Sur les figures, la référence 10 désigne la paroi du septum cardiaque, par exemple dans l'exemple décrit ci-dessous la paroi séparant la cavité 12 de l'oreillette gauche de la cavité 14 de l'oreillette droite, paroi dont l'épaisseur est de l'ordre de 2 mm.

On notera cependant que la description peut être transposée de façon semblable au perçage de la partie du septum cardiaque séparant le ventricule gauche du ventricule droit. En effet, comme on le comprendra à la lecture de la description ci-après, l'invention est applicable sans difficulté au perçage de parois relativement épaisses, ce qui est le cas du septum interventriculaire.

L'idée de base de l'invention consiste à utiliser comme outil de ponction la sonde de détection/stimulation elle-même, cette sonde étant une sonde à vis (vis fixe ou vis rétractable).

Plus précisément, comme illustré sur les Figures, cette sonde 16 comporte un corps de sonde de structure conventionnelle, avec une gaine 18 en matériau déformable, généralement une gaine en polyuréthanne ou silicone.

La sonde 16 est terminée à son extrémité proximale par un connecteur électrique 20 destiné à être couplé au boîtier du dispositif médical implanté (par exemple un stimulateur ou un resynchroniseur) après que la sonde ait été implantée au site de stimulation définitif choisi.

A son extrémité distale, la sonde est terminée par une tête de sonde 22 comprenant une vis hélicoïdale 24 à spires non jointives dont le diamètre est de l'ordre de 1 à 1,2 mm, et réalisée partiellement ou totalement en un matériau conducteur non isolé. Cette vis est électriquement reliée à un conducteur interne 26 par l'intermédiaire d'un embout métallique 28. Le conducteur 26 est par exemple un conducteur spiralé s'étendant sur toute la longueur de la gaine jusqu'au connecteur électrique 20, assurant ainsi la continuité électrique entre la vis hélicoïdale 24 et ce connecteur 20.

La gaine 18 peut être choisie pour procurer une certaine rigidité en torsion, de manière à pouvoir transmettre un couple de torsion depuis l'extrémité proximale de la sonde (au niveau du connecteur électrique) jusqu'à l'extrémité distale de la tête de sonde 22 afin d'entraîner en rotation la vis 24 pour la faire pénétrer par vissage dans le tissu cardiaque. Ce mouvement de rotation axiale est imprimé, selon le cas, soit directement au corps de sonde, soit à la fiche du connecteur pour une sonde de type *pin driven* (où côté proximal la fiche de connexion est solidaire d'un conducteur axial s'étendant à l'intérieur du corps de sonde, ce conducteur étant lui-même libre en rotation et relié à la vis hélicoïdale à son extrémité distale).

On notera que dans le cas d'une vis rétractable (au moyen d'un mécanisme connu), le mouvement de rotation imprimé au conducteur interne déploie simultanément la vis hors de son logement et assure sa pénétration dans la paroi du myocarde.

En variante ou en complément, si la rigidité en torsion du corps de sonde n'est pas suffisante, il est possible d'utiliser un mandrin de vissage spécifique 30 introduit dans la lumière interne de la sonde, l'extrémité de ce mandrin étant couplée à l'embout métallique 28 pour permettre l'entraînement en rotation de ce dernier, et donc de la vis 24, directement depuis l'extrémité proximale de la sonde.

Une sonde à vis, telle que la sonde 16 que l'on vient de décrire, est généralement utilisée comme sonde de détection/stimulation après ancrage de la vis au site de stimulation endocavitaire. Tel sera effectivement le cas dans le cadre de l'invention, le site de stimulation endocavitaire étant un site situé dans la cavité gauche 12, stimulé après traversée du septum 10 par la sonde 16.

L'idée de base de l'invention consiste à utiliser une telle sonde non seulement pour sa fonction de sonde de détection/stimulation - fonction qui sera quand même assurée *in fine* après traversée du septum -, mais également comme outil pour réaliser la ponction nécessaire pour réaliser le perçage préalable de la paroi du septum.

À cet effet, et de façon caractéristique de l'invention, la sonde 16 est reliée pendant la phase opératoire de perçage du septum à un générateur de ponction RF 32 dont la borne de sortie est alors couplée au connecteur électrique 20 - connecteur qui, dans sa fonction normale, est destiné à être ultérieurement relié au boîtier de l'implant stimulateur ou resynchroniseur.

On notera que la connexion du générateur RF 32 au connecteur 20 doit laisser la possibilité au médecin d'utiliser un mandrin de placement classique pendant la ponction, afin d'assurer en complément de la torsion un effort axial afin de maintenir la tête de la sonde sur la zone ponctionnée en complément de la traction exercée par la vis 24, de manière à sécuriser encore plus l'opération.

Le générateur 32 est un modèle connu, par exemple le *BMC Radio Frequency Perforation Generator* de Baylis Medical Company, Inc.

L'énergie RF produite par le générateur 32 est appliquée à la vis hélicoïdale 24 via le connecteur électrique 20, le conducteur spiralé 26 et l'embout métallique 28. Cette énergie va permettre la découpe des tissus dans la zone de très petite dimension définie par la vis hélicoïdale (dont le diamètre est, comme on l'a indiqué de l'ordre de 1 à 1,2 mm), et la rotation du corps de sonde va permettre de faire avancer à l'intérieur de la paroi du septum 10 la sonde au fur et à mesure de la progression de la ponction.

La partie distale 34 de la vis est isolée électriquement par un revêtement de parylène ou autre, par exemple sur l'étendue d'une spire ou sur une longueur de l'ordre de 0,8 mm en direction axiale. La partie proximale conductrice 36, non isolée, sera donc la seule partie électriquement active de la vis 24, ce qui permettra de concentrer localement le flux d'énergie RF dans la région correspondante, la partie distale 34 ne jouant qu'un rôle mécanique.

Grâce à cette configuration, la partie distale isolée 34 fait office d'organe mécanique de traction pour assurer la progression de la sonde dans le septum, tandis que la partie distale électriquement active 36 assure la découpe des tissus. Cette configuration est particulièrement adaptée aux parois épaisses telles que le septum interventriculaire, évitant notamment sur le long terme tout risque lié à la présence du corps de sonde définitif au travers de la valve mitrale.

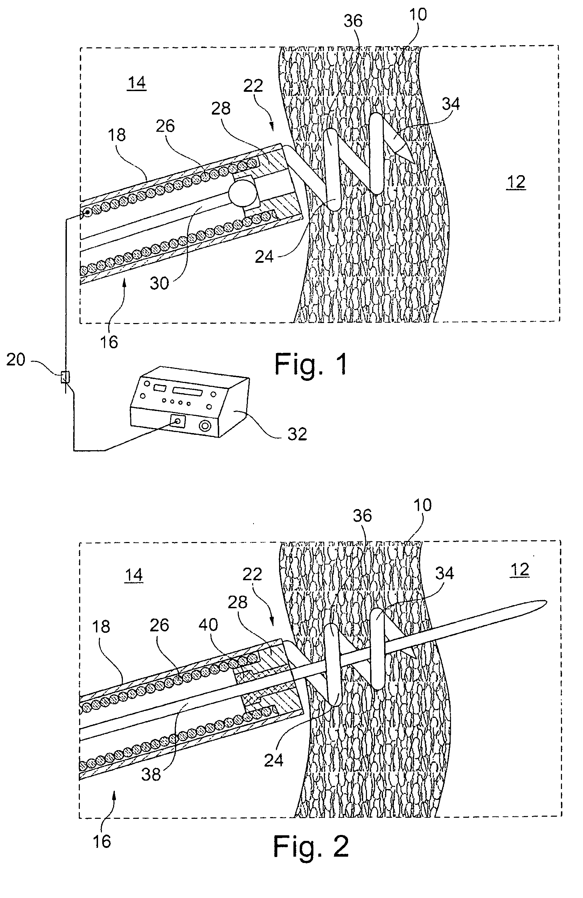

On va maintenant décrire le mode opératoire correspondant à la sonde illustrée Figure 1.

La première phase consiste à repérer le site de ponction, en manipulant l'extrémité de la sonde 16 par l'intermédiaire d'un mandrin classique introduit dans la sonde ou par un cathéter-guide, l'ensemble étant introduit dans la cavité 14 de l'oreillette droite jusqu'à venir en butée contre la paroi 10 du septum.

Une fois ce site atteint, le praticien imprime une rotation au corps de sonde et, par voie de conséquence, à la vis hélicoïdale 24, par l'intermédiaire de la gaine 18 et/ou du mandrin 30 depuis l'extrémité proximale de la sonde, au niveau du connecteur électrique 20. Cette manoeuvre va avoir pour effet de faire pénétrer la vis hélicoïdale 24 dans la paroi 10 du septum, le vissage complet étant détecté tactilement par le praticien du fait de la résistance opposée à la rotation.

L'étape suivante consiste à effectuer un test électrique et un examen radiographique selon différentes inclinaisons, pour confirmer le site de ponction choisi. Si la position n'est pas satisfaisante, le praticien peut dévisser la tête de sonde et déplacer celle-ci sous contrôle vers un autre point, puis tester le nouveau site.

Le connecteur électrique 20 est alors relié au générateur de ponction RF 32, qui est activé de manière à appliquer l'énergie RF au niveau de la vis 24, le retour de courant s'effectuant par une électrode de masse appliquée sur le corps du patient.

Tout en continuant à activer le générateur RF, le praticien maintient (via la gaine 18 et/ou le mandrin 30) la pression de la tête de sonde 22 contre la paroi du septum, et imprime au corps de sonde une rotation progressive contrôlée afin de faire progresser la vis hélicoïdale 24 dans la paroi 10 au fur et à mesure de l'avancement de la ponction.

Après que la paroi ait été complètement traversée, le générateur RF est arrêté et déconnecté de la sonde.

L'étape suivante consiste à pousser la sonde 16 au-delà de la paroi 10 du septum dorénavant traversé de part en part, jusqu'à ce que la vis hélicoïdale 24 aborde le site de stimulation endocavitaire choisi dans la cavité gauche 12 (oreillette ou ventricule), site où la tête de sonde sera définitivement ancrée en place par un dernier mouvement de vissage.

La Figure 2 illustre une variante de l'invention, basée sur la technique dite OTW (*Over The Wire*) ou de "filoguidage" au moyen d'un fil-guide très fin 38 pourvu à son extrémité distale d'une terminaison très souple, atraumatique.

Dans le cadre de la présente invention, le fil-guide 38 utilisé est un fil-guide RF, c'est-à-dire un fil-guide conducteur susceptible d'être relié au générateur de ponction RF 32. Le corps du fil-guide est isolé, et il n'est actif qu'à son extrémité distale, de manière à appliquer localement l'énergie RF pour réaliser une pré-ponction du septum sur un diamètre très faible.

Un fil-guide RF approprié est par exemple le modèle *Nykanen RF Wire* de la Société Baylis Medical Company, Inc., qui présente à son extrémité un diamètre réduit de 0,016 pouce (0,41 mm).

La sonde 16 utilisée avec un tel fil-guide est une sonde de type dit "porteuse", pourvue d'un joint d'étanchéité 40 à l'endroit de l'embout 28 à l'interface avec le fil-guide 38.

Une première forme de mise en oeuvre de cette variante consiste à introduire au préalable le fil-guide 38 puis à faire coulisser la sonde 16 sur ce fil-guide jusqu'au site choisi.

L'avantage de cette forme de mise en oeuvre réside dans la possibilité d'utiliser le fil-guide pour confirmer la zone ponctionnée : une fois la pré-ponction effectuée avec le fil-guide RF, il suffit de continuer à pousser ce fil-guide, qui aboutira facilement dans la cavité gauche 12 si la pré-ponction est correcte. L'autre avantage est de procurer un guidage supplémentaire de la sonde 16 pendant la durée de l'opération de ponction proprement dite (lors de l'application de l'énergie RF à la vis 24).

Une deuxième forme de mise en oeuvre consiste à poser en premier la sonde porteuse 16 sur la paroi, puis à insérer le fil-guide perceur 38 dans la porteuse et actionner le générateur RF. Le fil-guide passe alors dans l'oreillette gauche. Le médecin peut alors pousser le fil-guide 38 dans les cavités gauches, et confirmer par examen radiographique le site de ponction. C'est alors que le générateur RF est connecté à la sonde porteuse 16 (qui est également la sonde définitive) pour opérer la ponction finale de plus grand diamètre, la porteuse étant guidée par le fil-guide pendant cette ponction, ce qui constitue un autre avantage de cette variante.

L'invention présente de multiples avantages par rapport aux techniques connues.

Le premier avantage est la précision du site ponctionné, grâce au vissage préalable de l'embout de la sonde à vis, ce qui assure un ancrage précis de cette dernière, évitant toute coupe non maîtrisée susceptible d'entraîner de graves complications.

D'autre part, la manipulation ne fait appel qu'à des techniques proches de la pratique courante, suivant un accès classique par voie sous-clavière et avec des gestes familiers pour un praticien entraîné.

Enfin, un atout majeur d'une ponction RF opérée par la technique de l'invention réside dans le fait que l'effort à appliquer est indépendant de la nature ou de l'épaisseur des tissus.

Cet avantage est encore accru par la forme hélicoïdale de la vis, dont la progression naturelle dans les tissus par transmission d'un couple de rotation est contrôlable de façon très fine, bien mieux que l'avancée par pressions successives sur une aiguille comme cela est le cas avec les techniques de ponction RF classiques.

## Revendications

1. Un nécessaire comprenant, en combinaison :
- une sonde (16) de type sonde à vis, comportant :
· un corps de sonde avec une gaine (18) en matériau déformable ;
· côté proximale, un connecteur électrique (20) de couplage à un boîtier de dispositif médical implanté ;
· côté distal, une tête de sonde (22) avec une électrode comprenant une vis d'ancrage hélicoïdale saillante (24) au moins partiellement conductrice, apte à pénétrer dans une paroi du coeur (10) sous l'effet d'un mouvement de vissage imprimé à la tête de sonde depuis l'extrémité proximale de la sonde ; et
· un conducteur (26) s'étendant le long de la gaine, reliant audit connecteur électrique l'électrode comprenant ladite vis d'ancrage ; et
- un générateur de ponction radiofréquence (32), apte à être relié audit connecteur électrique pour permettre l'application contrôlée à la vis d'ancrage d'une énergie radiofréquence,
**caractérisé en ce que** ladite paroi du coeur (10) étant une paroi du septum cardiaque, la vis d'ancrage (24) comprend une partie distale de traction (34) électriquement isolée, prolongeant une partie proximale de découpe (36) électriquement conductrice apte à permettre l'application contrôlée à la vis d'ancrage de ladite énergie radiofréquence concurremment audit mouvement de vissage imprimé à la tête de sonde,
la sonde étant ainsi apte à assurer le perçage de ladite paroi jusqu'à traversée du septum par la tête de sonde pour permettre une implantation de la sonde par voie transseptale.

2. Le nécessaire de la revendication 1, dans lequel la sonde (16) est une sonde de détection/stimulation d'une cavité gauche du coeur, ladite électrode de la tête de sonde étant une électrode de détection/stimulation.

3. Le nécessaire de la revendication 1, dans lequel la vis d'ancrage est une vis fixe (24) prolongeant axialement la tête de sonde.

4. Le nécessaire de la revendication 1, dans lequel la vis d'ancrage est une vis mobile, rétractable dans un logement de la tête de sonde.

5. Le nécessaire de la revendication 1, dans lequel la gaine (18) du corps de sonde présente en torsion une rigidité insuffisante pour permettre la transmission sur toute sa longueur d'un mouvement de rotation imprimé depuis l'extrémité proximale de la sonde, et dans lequel le nécessaire comprend en outre :
un mandrin amovible (30), apte à être introduit dans une lumière du corps de sonde et mobile en translation à l'intérieur de cette lumière jusqu'à la tête de sonde et comportant des moyens de couplage en rotation avec la tête de sonde, ce mandrin présentant en torsion une rigidité suffisante pour permettre la transmission, sur toute sa longueur d'un mouvement de rotation imprimé depuis l'extrémité proximale du mandrin, pour le vissage de la vis d'ancrage.

6. Le nécessaire de la revendication 1, comprenant en outre :
- un fil-guide perceur radiofréquence (38) apte à permettre, après couplage au générateur de ponction radiofréquence, l'application contrôlée d'une énergie radiofréquence en un site de pré-ponction de la paroi du septum,
et dans lequel la sonde (16) est une sonde de type porteuse apte à être enfilée, sur ledit fil-guide.

## Claims

1. Kit comprising, in combination:
- a probe (16) of screw probe type, comprising:
• a probe body with a sheath (18) made of deformable material;
• proximal side, an electrical connector (20) for coupling to an implanted medical device module;
• distal side, a probe head (22) with an electrode comprising a protruding helical anchoring screw (24) that is at least partially conductive, capable of penetrating into a wall of the heart (10) under the effect of a screwing motion imparted on the probe head from the proximal end of the probe; and
• a conductor (26) extending along the sheath, linking the electrode comprising said anchoring screw to said electrical connector; and
- a radiofrequency puncture generator (32), which can be linked to said electrical connector to allow for the controlled application of a radiofrequency energy to the anchoring screw,
**characterized in that**, said wall of the heart (10) being a wall of the cardiac septum, the anchoring screw (24) comprises an electrically insulated pulling distal part (34), in extension of an electrically conductive cutting proximal part (36) which can allow for the controlled application to the anchoring screw of said radiofrequency energy concurrently with said screwing motion imparted on the probe head,
the probe thus being able to ensure the piercing of said wall until the probe head has passed through the septum to allow the probe to be implanted by transseptal pathway.

2. Kit according to Claim 1, in which the probe (16) is a probe for detection/stimulation of a left cavity of the heart, said electrode of the probe head being a detection/stimulation electrode.

3. Kit according to Claim 1, in which the anchoring screw is a fixed screw (24) in axial extension of the probe head.

4. Kit according to Claim 1, in which the anchoring screw is a mobile screw, which can be retracted into a recess of the probe head.

5. Kit according to Claim 1, in which the sheath (18) of the probe body exhibits, in torsion, a rigidity that is insufficient to allow for the transmission over its entire length of a rotational motion imparted from the proximal end of the probe, and in which the kit also comprises:
- a removable mandrel (30), which can be introduced into an opening of the probe body and which is mobile in translation inside said opening as far as the probe head and which includes means for rotational coupling with the probe head, this mandrel exhibiting in torsion a rigidity that is sufficient to allow for the transmission, over its entire length, of a rotational motion imparted from the proximal end of the mandrel, for the screwing of the anchoring screw.

6. Kit according to Claim 1, also comprising:
- a radiofrequency piercing guide wire (38) which can allow, after coupling to the radiofrequency puncture generator, for the controlled application of a radiofrequency energy at a pre-puncture site of the wall of the septum,
and in which the probe (16) is a bearer-type probe which can be threaded onto said guide wire.

## Patentansprüche

1. Besteck, das in Kombination enthält:
- eine Sonde (16) von der Art Schraubensonde, die aufweist:
• einen Sondenkörper mit einer Hülle (18) aus verformbarem Material;
• auf der proximalen Seite einen elektrischen Verbinder (20) zur Verbindung mit einem Gehäuse einer implantierten medizinischen Vorrichtung;
• auf der distalen Seite einen Sondenkopf (22) mit einer Elektrode, die eine vorstehende, zumindest teilweise leitende, wendelförmige Verankerungsschraube (24) enthält, die unter der Wirkung einer Schraubbewegung in eine Wand des Herzens (10) eindringen kann, die dem Sondenkopf vom proximalen Ende der Sonde aus verliehen wird; und
• einen sich entlang der Hülle erstreckenden Leiter (26), der die die Verankerungsschraube enthaltende Elektrode mit dem elektrischen Verbinder verbindet, die; und
- einen Radiofrequenz-Punktionserzeuger (32), der mit dem elektrischen Verbinder verbunden werden kann, um das kontrollierte Anlegen einer Radiofrequenzenergie an die Verankerungsschraube zu ermöglichen,
**dadurch gekennzeichnet, dass**, da die Wand des Herzens (10) eine Wand des Herzseptums ist, die Verankerungsschraube (24) einen elektrisch isolierten, distalen Zugbereich (34) hat, der einen elektrisch leitenden proximalen Schneidebereich (36) verlängert, der das kontrollierte Anlegen der Radiofrequenzenergie an die Verankerungsschraube zusammen mit der dem Sondenkopf verliehenen Schraubbewegung ermöglichen kann,
wobei die Sonde so in der Lage ist, das Durchbohren der Wand bis zur Durchquerung des Septums durch den Sondenkopf zu gewährleisten, um eine Implantierung der Sonde auf transseptalem Weg zu ermöglichen.

2. Besteck nach Anspruch 1, bei dem die Sonde (16) eine Erfassungs-/Stimulationssonde einer linken Höhle des Herzens ist, wobei die Elektrode des Sondenkopfs eine Erfassungs-/Stimulationselektrode ist.

3. Besteck nach Anspruch 1, bei dem die Verankerungsschraube eine ortsfeste Schraube (24) ist, die den Sondenkopf axial verlängert.

4. Besteck nach Anspruch 1, bei dem die Verankerungsschraube eine bewegliche Schraube ist, die in eine Aufnahme des Sondenkopfs zurückgezogen werden kann.

5. Besteck nach Anspruch 1, bei dem die Hülle (18) des Sondenkörpers eine unzureichende Torsionssteifigkeit aufweist, um die Übertragung einer Drehbewegung über ihre ganze Länge zu ermöglichen, die vom proximalen Ende der Sonde aus verliehen wird, und bei dem das Besteck außerdem enthält:
- einen entfernbaren Dorn (30), der in ein Langloch des Sondenkörpers eingeführt werden kann und im Inneren dieses Langlochs bis zum Sondenkopf translationsbeweglich ist und Drehverbindungseinrichtungen mit dem Sondenkopf aufweist, wobei dieser Dorn eine ausreichende Torsionssteifigkeit aufweist, um über seine ganze Länge die Übertragung einer Drehbewegung, die vom proximalen Ende des Dorns aus verliehen wird, zum Festschrauben der Verankerungsschraube zu ermöglichen.

6. Besteck nach Anspruch 1, das außerdem enthält:
- einen Radiofrequenz-Bohrführungsdraht (38), der nach der Verbindung mit dem Radiofrequenz-Punktionsgenerator das kontrollierte Anlegen einer Radiofrequenzenergie an einer Stelle der Vorpunktierung der Wand des Septums ermöglichen kann,
und bei dem die Sonde (16) eine Sonde von der Art Träger ist, die auf den Führungsdraht aufgeschoben werden kann.
